# EUROPEAN PATENT APPLICATION

(11) **EP 4 835 070 A1**
(43) Date of publication of application: **04.11.2026**
(21) Application number: 24911854.8
(22) Date of filing: 28.08.2024
(51) Int. Cl.: C12N 15/09, A01H 1/00, C12N 15/82, C12Q 1/6897, G16B 30/00, G16B 40/00

(54) **METHOD FOR PREDICTING PROMOTER ACTIVITY AND METHOD FOR MODIFYING PROMOTER BASED ON RESULTS OF PREDICTING PROMOTER ACTIVITY**

(30) Priority: 28.12.2023 JP 2023223673
(71) Applicant: GRA&GREEN Inc., Nagoya-shi, Aichi 464-0807 (JP)
(72) Inventor: TOYOKURA, Koichi, Nagoya-shi, Aichi 464-0807 (JP); TANIMOTO, Ren, Nagoya-shi, Aichi 464-0807 (JP); KONDO, Takayuki, Nagoya-shi, Aichi 464-0807 (JP)
(74) Representative: Plasseraud IP
(86) International application number: PCT/JP2024/030592
(87) International publication number: WO 2025/141959

(57) **Abstract**

The present disclosure provides a method for obtaining a promoter sequence modified so as to have a desired transcriptional activity. More specifically, there is provided a method for obtaining a promoter sequence modified so as to have a desired transcriptional activity, the method comprising providing an original promoter sequence to be modified, generating, based on the promoter sequence, a set of a plurality of modified promoter sequences that can be created by a genome editing technique, predicting, by a machine learning model, a transcriptional activity of each modified promoter sequence contained in the generated set of modified promoter sequences, and selecting a modified promoter sequence predicted to have the desired transcriptional activity.

## Description

### [TECHNICAL FIELD]

The present disclosure relates to prediction of promoter activity and modification of a promoter based on the prediction result thereof.

### [BACKGROUND ART]

At present, when a trait is imparted to a plant by genome editing, the main approach is a loss-of-function method in which a mutation is introduced into a coding region (CDS) and the function of the gene is disrupted by a frameshift.

On the other hand, methods may be contemplated in which functional enhancement is achieved by increasing the expression level of a target gene, or in which side effects of knockout are suppressed by leaving slight expression remaining rather than reducing expression to zero. Since studies in which function was enhanced by introduction of a transgene and knowledge obtained from functional analyses by RNAi can be applied, such methods are expected to greatly broaden the variation of traits that can be imparted by genome editing, and are important.

### [PRIOR ART DOCUMENTS]

### [NON-PATENT DOCUMENT]

[NON-PATENT DOCUMENT 1] Zhang, Yi, et al. "Applications and potential of genome editing in crop improvement." Genome Biology 19 (2018): 1-11.

### [SUMMARY OF THE INVENTION]

### [PROBLEMS TO BE SOLVED BY THE INVENTION]

As one of such methods, a method of editing not the CDS but a promoter region can be considered. Since regions such as promoters and enhancers determine the intensity of gene expression, it is considered that, by modifying the base sequence of such a region, the expression level can be increased or decreased while leaving the function of the gene itself unchanged.

In fact, the range (dynamic range) of gene expression level at the mRNA level as measured by the RNA-seq method is very large. While some mRNAs are contained in only about several copies, some mRNAs are contained in about 10⁵ copies, in a single cell. Such differences in gene expression level are considered to be brought about mainly by promoters and enhancers, and are considered to indicate the potential of a method of modifying the base sequence of a promoter region. Therefore, provision of a technique for precisely controlling gene expression level by genome editing is one of the objects according to the present disclosure.

### [MEANS FOR SOLVING THE PROBLEMS]

In regions such as promoters and enhancers, the correspondence relationship between sequence and function such as "this portion has this function," is more ambiguous than that in a CDS. Several cis-regulatory elements having highly conserved consensus sequences, as typified by the TATA box, have been discovered and databased. For example, software such as PromoterCAD of RIKEN and NEW PLACE of the National Agriculture and Food Research Organization is a system for searching for such cis-regulatory elements based on this knowledge and inferring whether a transcription factor binds to a promoter, but its accuracy is insufficient for the purpose of predicting expression level and making design.

Accordingly, the present inventors developed a system in which the relationship between base sequence and expression level is learned by machine learning, instead of a database system in which individual elements are registered. As a result, a high value of R² = 0.75 was obtained as a coefficient of determination indicating the correlation between measured values and predicted values. Then, sequences were designed using the prediction system, experiments using plants were conducted, and the accuracy of the prediction was demonstrated. If a certain base sequence can be given to a computer and transcriptional activity can be predicted, expression level can be "designed." As shown in the present disclosure, the present inventors demonstrated that the function (expression level) of a gene can be increased or decreased by editing the promoter of the gene based on prediction with a computer.

The present disclosure is based on these findings, and includes the following aspects:

### [Aspect 1]

A method for obtaining a promoter sequence modified so as to have a desired activity, the method comprising:
providing an original promoter sequence to be modified;
generating, based on the promoter sequence, a set of a plurality of modified promoter sequences that can be created by a genome editing technique;
predicting, by a machine learning model, an activity of each modified promoter sequence contained in the generated set of modified promoter sequences; and
selecting a modified promoter sequence predicted to have the desired activity.

### [Aspect 2]

The method according to Aspect 1, wherein the desired activity is a gene expression-inducing activity higher than that of the original promoter sequence, or a gene expression-inducing activity lower than that of the original promoter sequence.

### [Aspect 3]

The method according to Aspect 1, wherein the promoter sequence is a promoter sequence of a plant cell.

### [Aspect 4]

The method according to Aspect 1, wherein the original promoter sequence comprises a core promoter sequence and an upstream sequence thereof.

### [Aspect 5]

The method according to Aspect 1, wherein the genome editing technique is a genome editing technique using a CRISPR/Cas system.

### [Aspect 6]

The method according to Aspect 5, wherein the set of a plurality of modified promoter sequences is generated by sequence deletion caused by cleavage induced by a combination of guide RNA sequences designed based on two PAM recognition sequences.

### [Aspect 7]

The method according to Aspect 1, wherein the set of modified promoter sequences comprises at least 1,000 different sequences.

### [Aspect 8]

The method according to Aspect 1, wherein the machine learning model is a regression model trained to predict a gene expression-inducing activity from a promoter sequence, using, as training data, gene expression-inducing activity data of a plurality of promoter sequences in plant cells.

### [Aspect 9]

The method according to Aspect 1, further comprising visualizing, on a computer display, the activity of each modified promoter sequence contained in the generated set of modified promoter sequences.

### [Aspect 10]

An information processing apparatus for predicting a promoter sequence modified so as to have a desired activity, comprising:
a sequence input unit that receives input of an original promoter sequence to be modified;
a modified sequence generation unit that generates, based on the promoter sequence, a set of a plurality of modified promoter sequences that can be created by a genome editing technique;
an activity prediction unit that predicts, by a machine learning model, an activity of each modified promoter sequence contained in the generated set of modified promoter sequences; and
a sequence selection unit that selects a modified promoter sequence predicted to have the desired activity.

### [Aspect 11]

A non-transitory computer-readable medium storing instructions, wherein, when the instructions are executed by a processor, the instructions cause the processor to perform the steps of:
receiving input of an original promoter sequence to be modified;
   generating, based on the promoter sequence, a set of a plurality of modified promoter sequences that can be created by a genome editing technique;
predicting, by a machine learning model, an activity of each modified promoter sequence contained in the generated set of modified promoter sequences; and
selecting a modified promoter sequence predicted to have the desired activity.

### [Aspect 12]

A program for causing a computer to perform:
a function of receiving input of an original promoter sequence to be modified;
a function of generating, based on the promoter sequence, a set of a plurality of modified promoter sequences that can be created by a genome editing technique;
a function of predicting, by a machine learning model, an activity of each modified promoter sequence contained in the generated set of modified promoter sequences; and
a function of selecting a modified promoter sequence predicted to have the desired activity.

### [Aspect 13]

A method for genome editing of a cell for regulating an expression level of a desired gene, comprising:
obtaining a modified promoter sequence having a desired activity by the method according to Aspect 1;
providing a cell to be subjected to genome editing; and
editing the genome of the cell so as to generate the modified promoter sequence.

### [Aspect 14]

A method for producing a genome-edited plant in which an expression level of a desired gene is regulated, comprising:
editing the genome of a desired plant cell by the method according to Aspect 13; and
obtaining a plant individual derived from the genome-edited cell.

### [Aspect 15]

A machine learning model for predicting a gene expression-inducing activity in a plant cell from a promoter sequence, wherein the machine learning model is a regression model trained to predict a gene expression-inducing activity from a promoter sequence, using, as training data, gene expression-inducing activity data of a plurality of promoter sequences in a plant cell.

### [Aspect 16]

A method for generating a machine learning model for predicting a gene expression-inducing activity in a plant cell from a promoter sequence, comprising training a model using, as training data, gene expression-inducing activity data of a plurality of promoter sequences in a plant cell.

### [Aspect 17]

The method according to Aspect 16, wherein a transformer-based pre-trained foundation model is used as the model to be trained.

### [Aspect 18]

A method for obtaining a promoter sequence modified so as to have a desired activity, comprising:
providing an original promoter sequence to be modified;
generating, based on the promoter sequence, a set of a plurality of modified promoter sequences that can be created by a genome editing technique;
predicting, by a machine learning model, an activity of each modified promoter sequence contained in the generated set of modified promoter sequences; and
selecting a modified promoter sequence predicted to have the desired activity,
wherein the promoter sequence is a promoter sequence of a plant cell,
wherein the original promoter sequence comprises a core promoter and an upstream sequence thereof, wherein the core promoter is a sequence contained in positions -200 to +50 when the position of the base at the transcription start site is represented as +1,
wherein the genome editing technique is a genome editing technique using a CRISPR/Cas system,
wherein the set of a plurality of modified promoter sequences is generated by sequence deletion caused by cleavage induced by a combination of guide RNA sequences designed based on two PAM recognition sequences, wherein at least one PAM recognition sequence among the two PAM recognition sequences is located within the core promoter, and
wherein the original promoter has a length of at least 2,000 bp.

### [BRIEF DESCRIPTION OF THE DRAWINGS]

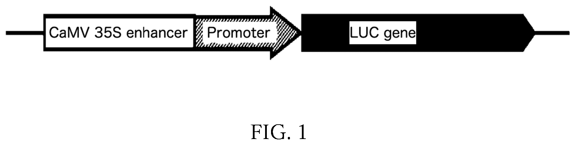

[FIG. 1] FIG. 1 is a schematic diagram showing the structure of artificial synthetic genes. Seven promoters were selected from a high-expression group, eight promoters were selected from a medium group, and four promoters were selected from a low-expression group, and artificial synthetic genes in which they were connected to a luciferase (LUC) gene were created. The promoters have 19 various sequence patterns. The sequence of the downstream LUC gene and the sequence of the upstream cauliflower mosaic virus (CaMV) 35S enhancer are common to each synthetic gene.

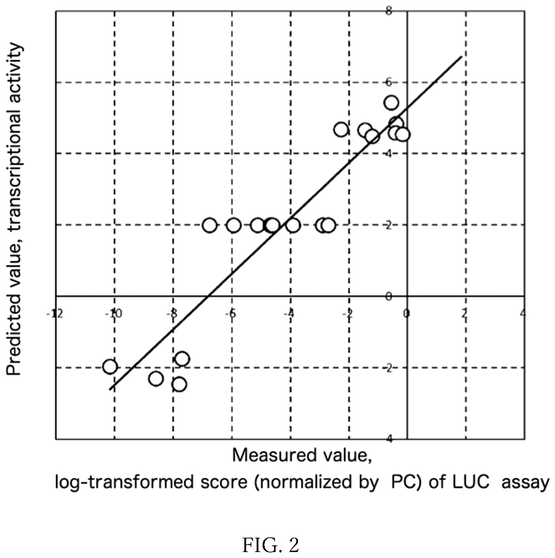

[FIG. 2] FIG. 2 is a scatter plot showing the relationship between measured values of the expression level of the LUC gene and predicted values of promoter strength. The vertical axis indicates predicted promoter strength. The higher the value, the greater the expression level of the downstream gene is expected to be. The horizontal axis indicates measured values of the LUC expression level. It should be noted that they were normalized by the transcriptional activity of the CaMV promoter used as a positive control.

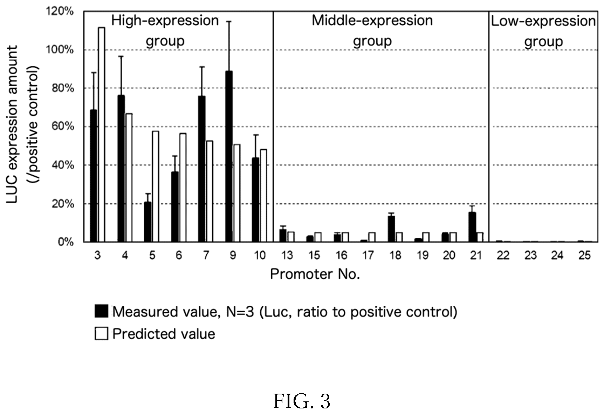

[FIG. 3] FIG. 3 shows results of calculating and comparing the LUC expression levels corresponding to the predicted transcriptional activities. The expression level of LUC was plotted on the vertical axis. It should be noted that they are shown as relative values with the emission intensity of the positive control set as 1. As the positive control, a cauliflower mosaic virus (CaMV) 35S promoter known to exhibit a high transcriptional activity was used. The numbers of the 19 promoters are shown on the horizontal axis. Promoter Nos. 3 to 10 were classified into a "high expression" group in which high expression strength was predicted, promoter Nos. 13 to 21 were classified into a "medium" group, and promoter Nos. 22 to 25 were classified into a "low expression" group. Black bar graphs indicate measured values of the LUC assay. White bar graphs indicate predicted values of transcriptional activity by the prediction system of the present disclosure.

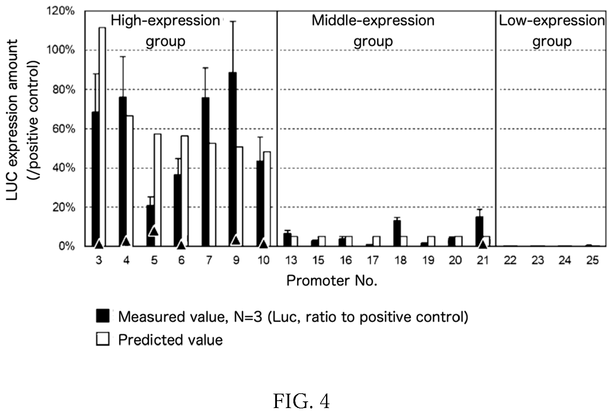

[FIG. 4] FIG. 4 shows results of searching for editing patterns capable of reducing transcriptional activity. For promoter Nos. 3, 4, 5, 6, 9, 10, and 21, editing patterns capable of reducing transcriptional activity were searched for. Black triangular plots indicated predicted values of the scores (expression levels) of the LUC assay calculated based on the predicted new transcriptional activities. As a result, transcriptional activities of about 14% to 1% relative to those of the original promoters were predicted.

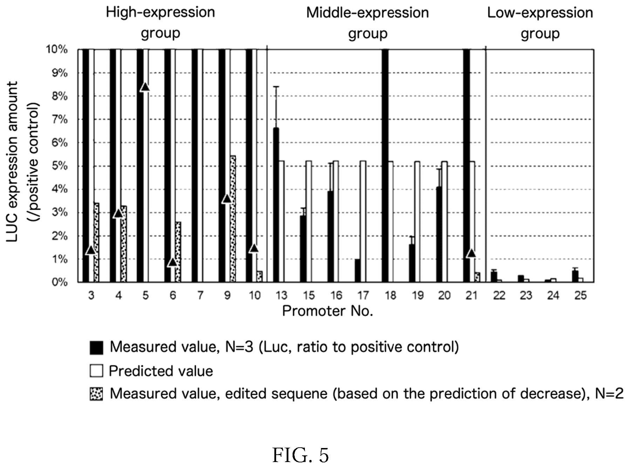

[FIG. 5] FIG. 5 shows, superimposed on FIG. 4, measured values of LUC expression levels of the new promoters as shaded bar graphs. Since the expression levels were very low, the vertical axis was shown on an enlarged scale. Promoter No. 5 was a missing value. For all six promoters for which measured values were obtained, marked decreases in expression level were observed, consistent with the predicted values.

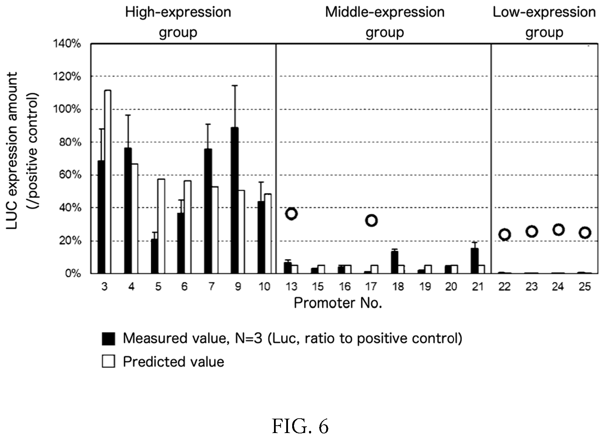

[FIG. 6] FIG. 6 shows results of searching for those whose predicted values of transcriptional activity increase after editing. For promoter Nos. 13, 17, 22, 23, 24, and 25, those showing increased transcriptional activity were selected, and predicted expression levels were shown by white circles. LUC expression levels of 7.4- to 125-fold relative to those of the original promoters were predicted.

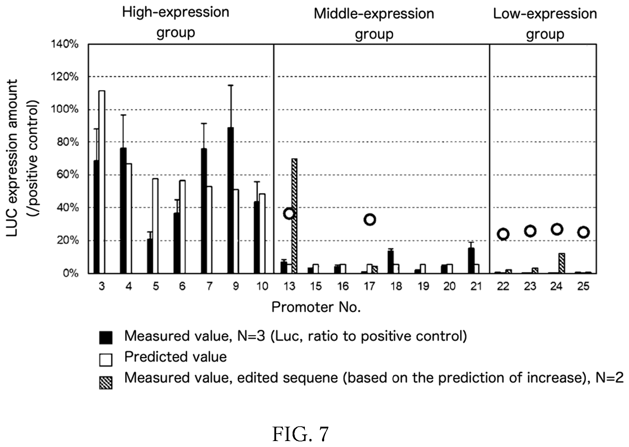

[FIG. 7] FIG. 7 shows results obtained by artificially synthesizing genes having theoretical promoter sequences, similarly transfecting protoplasts therewith, and measuring expression levels with a plate reader. Of the six promoters, expression levels of five promoters increased relative to those of the original promoters, but only No. 13 showed an increase in expression level exceeding the predicted value.

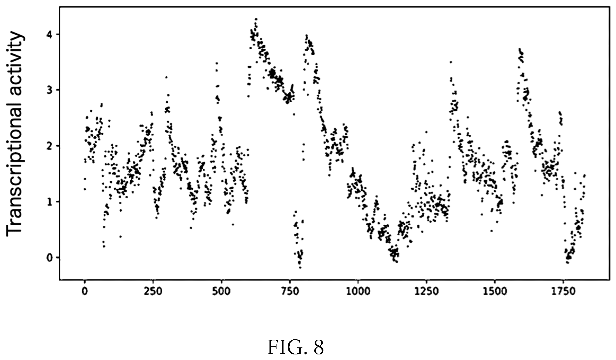

[FIG. 8] FIG. 8 shows an example of a visualization method for analyzing the entire picture of an input base sequence X. The vertical axis corresponds to predicted transcriptional activity, and the horizontal axis corresponds to positions in the base sequence.

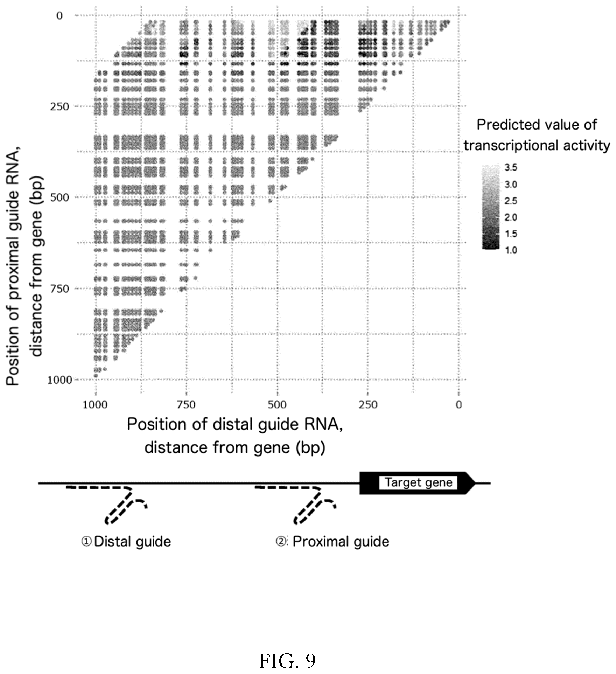

[FIG. 9] FIG. 9 shows an example of visualization by another method. The vertical axis corresponds to the position of guide RNA (proximal guide) designed at a position close to the target gene, and the horizontal axis corresponds to the position of guide RNA (distal guide) designed at a distant position. The color of the plot at each position is changed based on the estimated value of transcriptional activity. For example, according to a color chart, bright gray is for those estimated to have high transcriptional activity, and black is for those estimated to have low transcriptional activity.

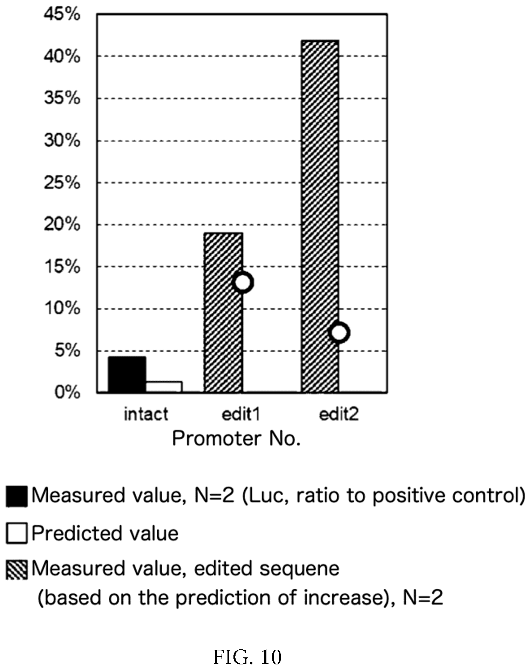

[FIG. 10] FIG. 10 shows results of an examination for increasing transcriptional activity with respect to a specific soybean gene. Based on the promoter of a soybean gene, two promoter sequences after genome editing for increasing gene expression were created (edit1 and edit2). The transcriptional activity of edit1 was predicted to be 2.950119. Further, the LUC expression level was predicted by linear regression to be 13.2% relative to the P control. The measured value of the LUC assay of this promoter was 19.0% relative to the P control. The transcriptional activity of edit2 was predicted to be 2.432977. Further, the LUC expression level was predicted to be 7.27% relative to the P control. The measured value of the LUC assay of this promoter was 41.6% relative to the P control.

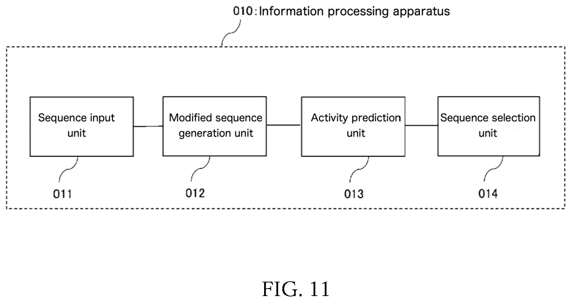

[FIG. 11] FIG. 11 shows an exemplary configuration of an information processing apparatus for predicting a promoter sequence modified so as to have a desired activity.

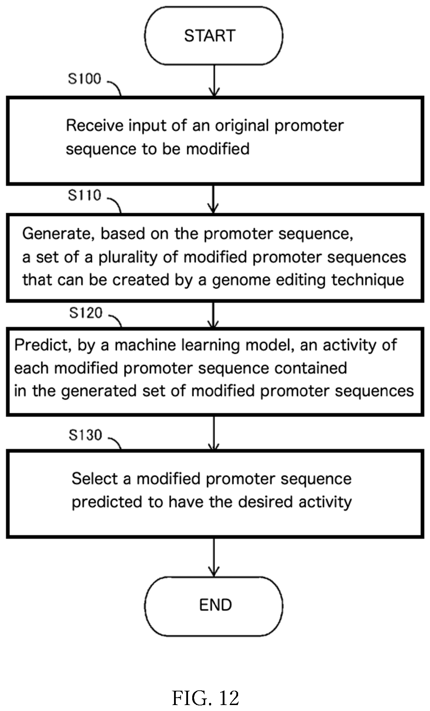

[FIG. 12] FIG. 12 shows an exemplary flowchart of a program for causing a computer to perform a function of receiving input of an original promoter sequence to be modified, a function of generating, based on the promoter sequence, a set of a plurality of modified promoter sequences that can be created by a genome editing technique, a function of predicting, by a machine learning model, an activity of each modified promoter sequence contained in the generated set of modified promoter sequences, and a function of selecting a modified promoter sequence predicted to have the desired activity.

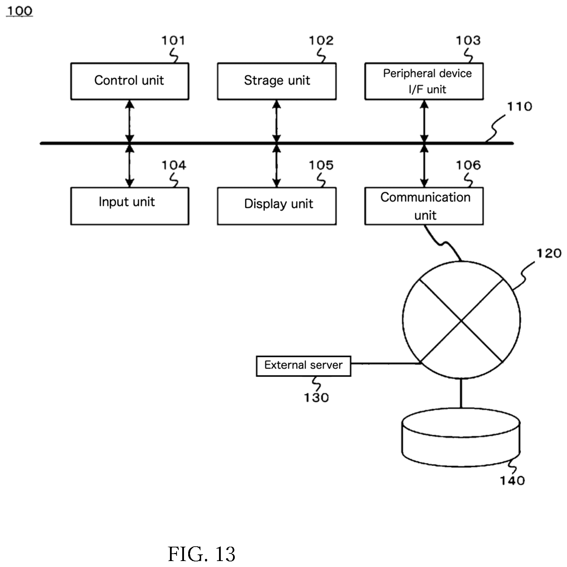

[FIG. 13] FIG. 13 is a schematic configuration of a computer that can be used to implement aspects of the present disclosure.

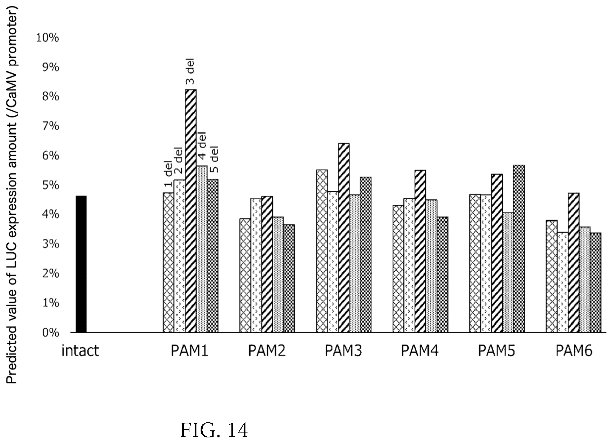

[FIG. 14] FIG. 14 shows results of in silico genome editing of the promoter sequence of the AT4G39130 gene of Arabidopsis thaliana with one target (one guide RNA).

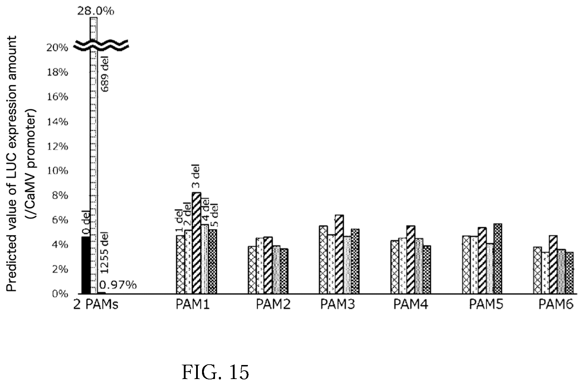

[FIG. 15] FIG. 15 shows a clear difference in the change generated in gene expression level between a case in which deletion at two targets was performed in a promoter region and a case in which deletion at one target was performed.

### [MODE FOR CARRYING OUT THE INVENTION]

In the course of crop breeding, breeders have a desire to enhance or suppress expression of a particular gene. As gene functions have come to be analyzed, relationships between genes and traits have been successively elucidated. In the example of semidwarfism in rice, semidwarfism was regarded as a breeding target in rice, and in 1966 the semidwarf variety "Reimei" was bred. In semidwarf rice, even when fertilizer is applied, plant height is less likely to increase, and yield does not decrease. When fertilizer is applied, ordinary rice grows vertically, but this causes the rice to lodge easily due to typhoons or strong winds, making harvesting impossible or lowering quality. Therefore, fertilization beyond a certain level causes lodging and does not lead to an increase in harvest yield. Since semidwarf rice is less likely to increase in plant height, it is resistant to lodging even when a large amount of fertilizer is applied, and yield increased. It is now clear that the mutation that imparted semidwarfism to Reimei is present in a gene encoding a G20 oxidase in the gibberellin biosynthetic pathway. Semidwarf crops were a great invention that brought tremendous progress to the expansion of food production.

Such relationships between genes and traits have been successively elucidated, and knowledge has been accumulated. As a result, target genes have often come to be set in order to obtain a desired trait. For example, when semidwarfism is to be introduced into rice, it is sufficient to select a mutant of the above gene (sd1 mutant). Although it is possible to search for naturally occurring mutants, mutations may also be introduced randomly by chemical treatment or irradiation. Although the loss-of-function allele of sd1 is considered to be recessive, an individual in which both alleles are loss-of-function sd1 can be bred by planned crossing.

With the advent of genetic recombination technology, it became possible to artificially design genotypes. That is, by introducing a certain gene sequence into a crop of interest, a function or trait can be imparted thereto. A representative example is herbicide-tolerant corn (Roundup Ready^{®}). Roundup^{®} (glyphosate) is an herbicide and is, in substance, an amino acid analog. In plants and some bacteria, when glyphosate is taken up, amino acid synthesis is inhibited and the organism dies. By introducing, from bacteria, a gene that detoxifies glyphosate, tolerance to glyphosate was imparted to plants, and this was a great invention that dramatically reduced the cost of weeding. On the other hand, production and use of genetically modified plants into which foreign genes are introduced has been limited, and all crops on earth have not been replaced by genetically modified crops. In particular, opposition has been strong in Europe, and even now cultivation of genetically modified crops is greatly restricted in Europe, with only one cultivated variety (corn in Spain).

Since genome editing was invented, the situation has changed further. It has become possible, without genetic modification, to introduce a mutation directly into a target gene. This can be said to be a historic invention. For example, when semidwarfism is to be introduced into Koshihikari, it is sufficient to induce a frameshift by introducing a small indel of about 1 to 10 bases into the SD1 locus and thereby disrupt its function. By this method, development can be achieved earlier than by chemical- or radiation-induced mutagenesis (and subsequent backcrossing about 5 to 7 times), and unintended trait expression due to linkage drag can be avoided, thus providing a great advantage in terms of cost. An example of food products created by this method and sold in Japan is red sea bream sold by Regional Fish Co., in which the myostatin gene is disrupted.

In genome editing, in addition to small indels of about 1 to 10 bases, medium-scale deletions of up to several thousand bases can also be induced. In this case, two guide RNAs are delivered simultaneously into a cell to cleave two target sequences in DNA, and when the two cleavage sites are linked or otherwise physically close, the region between the two cleavage sites may drop out and be repaired. A specific example is waxy corn of Corteva Agriscience, in which approximately 4 kb including the entire coding sequence of the Wx locus is deleted.

The mutations in the above-mentioned varieties bred via genome editing are of a type that can also occur in nature, and the resulting products (crops) are not substantially different from breeding by conventional crossing. In contrast, homologous recombination can also be caused using genome editing, whereby a target position in the genome can be replaced with a certain DNA sequence. By applying this technique, various mutations such as point mutations, deletions, and insertions can be introduced, and furthermore genetic recombination can also be caused by replacement with an exogenous gene. Thus, although referred to collectively as genome editing, it is possible to create mutants belonging to essentially different categories, such as mutations that can occur in nature and genetic recombinants that cannot occur at all under natural conditions. In order to avoid confusion, varieties created by genome editing have been classified into three categories, SDN1 to SDN3. Both the example of Regional Fish Co. and the example of Corteva described above are classified as SDN1. For genome-edited crops, procedures such as notification to administrative agencies and prior consultation are required in many countries including Japan when a new variety is created. In SDN1, since the newly introduced mutation is essentially equivalent to a naturally occurring mutation and precedents have also accumulated, procedures are expected to proceed rapidly in many countries, which is industrially advantageous.

It has been described above that a frameshift can be induced by genome editing, thereby making it possible to readily create a loss-of-function mutant of a specific gene. However, for breeders, loss-of-function mutants of genes are not the only useful ones. Mutants accompanied by increased gene expression, or mutants accompanied by decreased gene expression, have been selected in countless numbers, whether intentionally or unintentionally. In addition, an enormous number of gene silencing (RNAi, KD) experiments have been reported in the history of plant science. Phenotypes may differ between knockouts (KO) and knockdowns (KD). It is considered that there are also cases in which retention of slight expression avoids a harmful phenotype. Thus, a marked decrease in gene expression level that mimics a KD is also often considered useful. The method according to the present disclosure has the advantage of making it possible to design such increases or decreases in gene expression level by using SDN1 genome editing, which can be commercialized in Japan only through a process of notification to the relevant ministries and agencies.

As a method for enhancing function, one conceivable method is to introduce a mutation into a gene. For example, it is considered that activity can be enhanced by disrupting an autoinhibitory domain of an enzyme. Taking a crop sold in Japan as an example, in the high-GABA accumulating tomato of Sanatech Seed Co., GABA synthetic activity is enhanced by disrupting, by frameshift, the autoinhibitory domain present at the C-terminus of GAD, a GABA synthase. This method is very effective, but can be used only when the target protein (gene) has an autoinhibitory domain. Since proteins having an autoinhibitory domain are relatively few in number, the range of application is narrow. As other methods, it is conceivable to disrupt the localization signal of a protein so that it is always in an active form, to replace an amino acid to be phosphorylated with glutamic acid, or to improve reactivity by introducing a mutation into the active site of an enzyme. However, such methods cannot generally be used for any gene.

As another method for enhancing function that is generally applicable to many genes, a method may be contemplated in which a mutation is introduced into a region that regulates the expression level of a gene. That is, the idea is that expression level is increased by editing a promoter and function is thereby enhanced, but this idea has a drawback which is that, even in a promoter, the correspondence relationship between sequence and function is ambiguous. In genes, the correspondence relationship between codons and amino acid sequences is strict, and functional domains formed by specific amino acid sequences are also highly conserved, and therefore function can be inferred from sequence. For this reason, disrupting a target domain can be carried out with a high degree of certainty. In contrast, in promoters (non-ORFs), it is difficult to formulate a hypothesis such as "if this portion is changed in this way, such-and-such will occur."

Of course, a relationship between sequence and function also exists. Cis-regulatory elements typified by the TATA box are understood as binding sites for basal transcription factors and transcription factors, and there are cases in which transcription factors have been successfully inferred from sequence and binding sites have been successfully inferred from transcription factors. For example, web applications such as PromoterCAD of RIKEN and NEW PLACE of the National Agriculture and Food Research Organization are tools for searching for such elements. Although such tools have been proposed, examples in which breeding has been successfully achieved using them are considered to be limited. Song et al., 2022 reported enhancement of gene expression by genome editing of a promoter (Song, X., Meng, X., Guo, H. et al. Targeting a gene regulatory element enhances rice grain yield by decoupling panicle number and size. Nat Biotechnol 40, 1403-1411 (2022). https://doi.org/10.1038/s41587-022-01281-7). In this report, it was reported that increased yield could be obtained by editing the promoter or 5'UTR of the rice IPA1 gene. The procedure is to create lines having various deletion patterns by genome editing, evaluate expression patterns and traits, and identify an important regulatory element. This shows how difficult it is to design function from a regulatory element. Fluctuation of regulatory elements (typically, permitting mismatches of a few bases from a consensus sequence) is one reason for the difficulty. Recently, there have also been reports in which cis-regulatory elements are inferred by application of deep learning, but there has been absolutely no example in which such inference has actually led to a crop trait.

The present inventors adopted a strategy of directly inferring expression level from a DNA sequence instead of the conventional method of inferring function from cis-regulatory elements. For that purpose, the target was limited to promoters. Although the relationship between sequence and expression level is a black box, methods using deep learning often exhibit high performance even when the principle or theory is unclear. For example, in the field of machine translation, until the 1990s, methods were explored in which vocabularies were collected, dictionaries linking words to functions (semantic information) were constructed, and languages were converted (for example, conversion from English to German), but high performance was not obtained. In contrast, around 2011, when WATSON created by applying a neural network (NN), exhibited high performance in natural language processing, it became possible to realize translation with high accuracy without inputting the meaning of individual sentences, by training on a vast amount of text data, rather than by having humans create dictionaries and causing computers to understand meanings. Google Translate and DeepL are representative examples of machine translation using NNs at present, and both provide very high utility. Thus, machine learning using NNs may exhibit high performance without humans understanding or organizing the principle or theory.

DNABERT is a model pre-trained to handle DNA sequences with reference to the natural language model BERT created for the purpose of natural language processing. DNABERT was developed for the purpose of analyzing non-coding regions based on DNA sequences, and has been shown to exhibit high performance in tasks such as discovery of promoters and splice sites. For this reason, it was decided to use DNABERT for the problem of the present invention, namely, predicting the transcriptional activity of a core promoter. However, in applying DNABERT to the present invention, there were two problems.

First, since the human genome was used as pre-training data, it was unclear whether plant genomes could be handled by DNABERT. In plants, as core elements constituting a core promoter, there may be plant-specific elements such as Y patch, CA, and GA, in addition to factors common to animals such as the TATA box, Initiator, and Kozak. In addition, in promoters of animal and plant genes, transcriptional activity may be regulated by methylation of DNA. In animals, cytosine in a sequence called CG (CpG) is in most cases methylated; in contrast, in plants, cytosine in non-CG sequences is also methylated. Thus, although the basic mechanism of transcription is common between animals and plants, the constituent elements such as core elements and methylation sites differ. Accordingly, in plants having promoters whose structure greatly differs from that of humans, it was unknown whether DNABERT would exhibit high performance.

Second, although DNABERT had been shown to exhibit high performance in tasks such as discovery of transcription start sites and splice sites, these were problems of determining whether an element of interest "is present" or "is absent" in a given sequence, and were binary classification problems. In contrast, the problem addressed by the present inventors was to predict the expression intensity of a gene downstream of a core promoter sequence, and output of a continuous value was required. It was entirely unknown whether DNABERT would be effective for such a regression problem and whether it would exhibit high performance. When handling continuous values related to DNA sequences, it had been common to use CNN. Based on reports that regression problems can also be handled by applying the natural language processing model BERT, the present inventors modified the DNABERT code. Specifically, the code was modified by adding a class so that continuous values could be handled.

Despite such problems, the present inventors succeeded, by fine-tuning the pre-trained model of DNABERT according to the procedure separately shown, in predicting promoter strength with high accuracy. Furthermore, the inventors also succeeded in developing a program incorporating this learning model and changing promoter function by genome editing.

In one aspect, the present disclosure relates to a method for obtaining a promoter sequence modified so as to have a desired activity. In some embodiments, the method according to the present disclosure may be a method comprising providing an original promoter sequence to be modified, generating, based on the promoter sequence, a set of a plurality of modified promoter sequences that can be created by a genome editing technique, predicting, by a machine learning model, the activity of each modified promoter sequence contained in the generated set of modified promoter sequences, and selecting a modified promoter sequence predicted to have the desired activity.

A promoter is a DNA sequence located (normally) upstream of a gene, is not itself transcribed, but has the function of determining the transcription amount (expression level) of the gene and, in multicellular organisms, the site of expression and tissue specificity. In the present specification, in particular, a region in the vicinity of a gene that interacts with basal transcription factors is referred to as a "core promoter." A core promoter may be defined as positions -200 to +50 when the position of the base at the transcription start site is represented as +1. More preferably, it may be defined as positions -180 to +25. Still more preferably, it may be defined as positions -170 to +17. Most preferably, positions -165 to +5 may be defined as the core promoter. Alternatively, the position of the core promoter may be defined as a range having, as both ends thereof, any one of positions -200, -195, -190, -185, - 180, -175, -170, and -165, and any one of positions +5, +8, +12, +17, +25, +30, +40, and +50. The core promoter is considered to have the function of determining a basic transcription amount. The length of the core promoter may be, for example, about 250 bp, about 225 bp, or about 200 bp.

In some embodiments, the promoter sequence is a promoter sequence of a plant cell. It is known that, in animals and plants, the types of core elements contained in a core promoter differ. In the present specification, the "plant" is not particularly limited. For example, a wide range of plants can be mentioned, including bryophytes, pteridophytes, gymnosperms, magnoliids, monocots, and eudicots (rosids I, rosids II, asterids I, asterids II, and outgroups thereof). More specific examples of plants include solanaceous plants such as tomato, sweet pepper, chili pepper, eggplant, tobacco, and torvum; cucurbits such as cucumber, pumpkin, melon, and watermelon; brassica vegetables such as cabbage, broccoli, Chinese cabbage, and kale; leafy and spice vegetables such as perilla, celery, parsley, and lettuce; alliums such as Welsh onion, onion, and garlic; other fruit vegetables such as strawberry and melon; taproot crops such as radish, turnip, carrot, and burdock; tuber crops such as taro, cassava, potato, sweet potato, and Chinese yam; cereals such as rice, corn, wheat, sorghum, barley, rye, Brachypodium distachyon, and buckwheat; legumes such as soybean, adzuki bean, mung bean, cowpea, common bean, peanut, pea, and broad bean; soft vegetables such as asparagus, spinach, and Japanese honewort; flowering plants such as lisianthus, rose, stock, carnation, and chrysanthemum; turfgrasses such as bentgrass and zoysiagrass; oil crops such as rapeseed, camelina, Brassica napus, Jatropha curcas, sesame, and perilla; fiber crops such as cotton, rush, and hemp; forage crops such as clover, dent corn, and alfalfa; deciduous fruit trees such as apple, pear, grape, and peach; citrus plants such as Satsuma mandarin, orange, lemon, and grapefruit; and woody plants such as satsuki azalea, azalea, cedar, poplar, and Pará rubber tree. The promoter may also be site-specific or non-site-specific. A site-specific promoter may, for example, control specific expression in leaves or roots.

In some embodiments, the desired activity required for the modified promoter may be a gene expression-inducing activity higher than that of the original promoter sequence, or a gene expression-inducing activity lower than that of the original promoter sequence. The gene expression-inducing activity higher than that of the original promoter sequence may be, for example, any of 1.1-fold to 1,000-fold of the original activity, for example, 1.1-fold, 1.2-fold, 1.5-fold, 2-fold, 3-fold, 4-fold, 5-fold, 10-fold, 20-fold, 50-fold, 100-fold, 200-fold, 300-fold, 400-fold, 500-fold, 600-fold, 700-fold, 800-fold, 900-fold, or 1,000-fold. The gene expression-inducing activity lower than that of the original promoter sequence may be, for example, any of 90% to 0.01% of the original activity, for example, 90%, 80%, 50%, 10%, 5%, 1%, 0.5%, 0.1%, 0.05%, 0.04%, 0.03%, 0.02%, or 0.01%.

In some embodiments, the desired activity required for the modified promoter may be determined by comparison not only with the activity of the original promoter sequence but also with the activity of another reference promoter sequence.

Alternatively, the desired activity required of the modified promoter may be determined by stratification such as high expression, medium expression, and low expression. In some embodiments, stratification of any number or range may be carried out.

In some embodiments, modification of the promoter sequence may be that by deletion, substitution, or insertion of a part of the sequence. In some embodiments, modification of the promoter sequence may be deletion of a part of the sequence by cleavage at two sites in the promoter sequence.

In some embodiments, the original promoter sequence to be modified may be prepared by obtaining the sequence from a database such as GenBank. Examples of the promoter sequence include any DNA region including the transcription start site within the DNA region from -9,900 to +100, preferably from -4,950 to +50 or from -2,975 to +25, more preferably from -1,995 to +5, around the transcription start site of the target gene. Alternatively, any DNA region between the transcription start site of the target gene and the transcription termination site of an adjacent gene may be used. That is, as the promoter sequence, a region including the core promoter and an upstream sequence thereof may be used. The length of the sequence upstream of the core promoter may be, for example, at least 200 bp, 400 bp, 600 bp, 800 bp, 1,000 bp, 1,200 bp, 1,400 bp, 1,600 bp, 1,800 bp, 2,000 bp, 3,000 bp, 4,000 bp, 5,000 bp, 6,000 bp, 7,000 bp, 8,000 bp, or 9,000 bp. Further, the length of the sequence upstream of the core promoter may be, for example, 15,000 bp or less, 12,500 bp or less, or 10,000 bp or less. When there are a plurality of transcription start sites, the plurality of transcription start sites may be selected. Further, for example, any range of 100 to 1,800 bases contained in positions -1,995 to +5 around the transcription start site may be used. The length of the promoter (number of base pairs: bp) may be, for example, at least 200 bp, 400 bp, 600 bp, 800 bp, 1,000 bp, 1,200 bp, 1,500 bp, 2,000 bp, 3,000 bp, 4,000 bp, 5,000 bp, 6,000 bp, 7,000 bp, 8,000 bp, or 9,000 bp, and may range, for example, from 100 to 10,000 bp, preferably from 200 to 5,000 bp, more preferably from 500 to 3,000 bp.

In some embodiments, in order to select a modified promoter sequence having a desired activity, a set of a plurality of modified promoter sequences that can be created by a genome editing technique is generated based on the promoter sequence.

As the genome editing technique, ZFNs, TALENs, and CRISPR-Cas systems may be used. Examples of the CRISPR-Cas system include Class 1 Type I CRISPR-Cas3, Class 2 Type II Cas9, Class 2 Type V Cas12a, Class 2 Type V Cas12f (Cas14a), and Class 2 Type VI Cas13a, and the functional mechanism and classification thereof are not particularly limited. Further, Cas proteins derived from various bacteria may be used. For example, examples of Cas9 include SpCas9 derived from *Streptococcus pyogenes*, SaCas9 derived from *Staphylococcus aureus*, FnCas9 derived from *Francisella novicida*, and CjCas9 derived from *Campylobacter jejuni*, and examples of Cas12a include AsCas12a (AsCpf1) derived from *Acidaminococcus* sp., LbCas12a (LbCpf1) derived from *Lachnospiraceae bacterium*, and ErCas12a derived from *Eubacterium rectale*, and the origin thereof is not limited. Further, there may also be used Cas proteins in which the base sequence encoding the Cas protein or the amino acid sequence of the Cas protein has been modified, Cas proteins fused with another protein, functional domain, peptide, or amino acid sequence, and Cas proteins modified with a compound.

In the CRISPR-Cas system, a Cas nuclease and a guide RNA (gRNA) are used for cleavage of the target sequence. The guide RNA consists of two elements, crRNA and tracrRNA, and these may exist as separate RNAs or may be linked and exist as a single-stranded RNA. A single-stranded guide RNA is also referred to as an sgRNA. To the 5' end, 3' end of the guide RNA, a base sequence of 1 base to 10 bases, 10 bases to 50 bases, 50 bases to 100 bases, or 100 bases to 500 bases may be added. The guide RNA binds complementarily to a specific DNA sequence and guides the Cas nuclease to a specific position in the genome. As a result, the Cas nuclease cleaves the specific DNA sequence and enables genome editing. For example, when CRISPR is used, the PAM sequences in the promoter sequence are searched and a list of cleavable positions is prepared. Depending on the Cas protein used, examples of the PAM sequence include NGG for SpCas9, NGRRT or NGNRRN for SaCas9, NNNNGATT for NmeCas9, NNNNRYAC for CjCas9, TTTV for LbCas12a (Cpf1), TTTV for AsCas12a (Cpf1), TTN for AacCas12a (Cpf1), and ATTN, TTTN, or GTTN for BhCas12b v4, but when the sequence is one that can be recognized by the Cas protein, these PAM sequences may also include 1-base, 2-base, 3-base, 4-base, 5-base, 6-base, 7-base, or 8-base mismatches. The presence of a PAM sequence is important for increasing the accuracy and specificity of genome editing in the CRISPR system. In the absence of a PAM sequence, the Cas nuclease cannot bind to the DNA sequence specified by the guide RNA. This property makes it possible to accurately target a specific gene region for genome editing. Further, as described above, by using different types of Cas nucleases, regions having different PAM sequences can also be targeted. Typically, for example, in the case of Cas9, a position 3 to 4 bases from the PAM sequence serves as the cleavage position, and in the case of Cas12a, a position 18 to 23 bases from the PAM sequence serves as the cleavage position. A person skilled in the art can design an appropriate guide RNA based on the PAM sequence in the target genome. Typically, about 50 cleavage positions are found within 2,000 bases. For example, the number of combinations in which two positions are designated in n cleavage positions is ₙC₂. That is, for 50 cleavage positions, there may be 1,250 combinations. Accordingly, in some embodiments, in order to select a modified promoter sequence having a desired activity, with respect to the ₙC₂ combinations of PAM sequences in the promoter sequence to be modified, a set of base sequences may be generated in which the region between two cleavage positions is trimmed (deleted). Thus, in some embodiments, the set of a plurality of modified promoter sequences is generated by sequence deletion caused by cleavage induced by a combination of guide RNA sequences designed based on two PAM recognition sequences. In some embodiments, at least one PAM recognition sequence of the two PAM recognition sequences may be located within the core promoter. In some embodiments, both of the two PAM recognition sequences may be located within the core promoter. In some embodiments, only one of the two PAM recognition sequences may be located within the core promoter. In some embodiments, the length of the sequence deletion caused by cleavage induced by the combination of guide RNA sequences designed based on the two PAM recognition sequences is not particularly limited as long as it is a sequence deletion (Large Deletion) larger than the deletion size caused by one target (one guide RNA) (deletion of a 1- to 5-base sequence), and may be, for example, in the range of 50 bp to 15,000 bp, preferably in the range of 50 bp to 5,000 bp. Therefore, in some embodiments, the length of the sequence deletion may be, for example, at least 50 bp, 75 bp, 100 bp, 150 bp, 200 bp, 150 bp, 200 bp, 250 bp, 300 bp, 350 bp, 400 bp, 450 bp, 500 bp, 600 bp, 700 bp, 800 bp, 900 bp, 1,000 bp, 1,100 bp, 1,200 bp, 1,300 bp, 1,400 bp, 1,500 bp, 1,600 bp, 1,700 bp, 1,800 bp, 1,900 bp, 2,000 bp, 2,100 bp, 2,200 bp, 2,300 bp, 2,400 bp, 2,500 bp, 3,000 bp, 4,000 bp, 5,000 bp, 6,000 bp, 7,000 bp, 8,000 bp, 9,000 bp, 10,000 bp, 11,000 bp, 12,000 bp, 13,000 bp, 14,000 bp, or 15,000 bp, preferably at least 400 bp. Further, in some embodiments, the length of the sequence deletion caused by cleavage induced by the combination of guide RNA sequences designed based on the two PAM recognition sequences may be 15,000 bp or less, 14,000 bp or less, 13,000 bp or less, 12,000 bp or less, 11,000 bp or less, 10,000 bp or less, 9,000 bp or less, 8,000 bp or less, 7,000 bp or less, 6,000 bp or less, 5,000 bp or less, 4,000 bp or less, 3,000 bp or less, 2,500 bp or less, 2,250 bp or less, 2,000 bp or less, 1,000 bp or less, or 500 bp or less. Further, in some embodiments, the length of the sequence deletion caused by cleavage induced by the combination of guide RNA sequences designed based on the two PAM recognition sequences may be any length between 400 bp and 3,000 bp, for example, 400 bp, 450 bp, 500 bp, 600 bp, 700 bp, 800 bp, 900 bp, 1,000 bp, 1,100 bp, 1,200 bp, 1,300 bp, 1,400 bp, 1,500 bp, 1,600 bp, 1,700 bp, 1,800 bp, 1,900 bp, 2,000 bp, 2,100 bp, 2,200 bp, 2,300 bp, 2,400 bp, or 2,500 bp. Further, in some embodiments, at least one of the two PAM recognition sequences may be located within 200 bases from the position of the base at the transcription start site, for example, within 190 bases, 180 bases, 170 bases, 160 bases, 150 bases, 140 bases, 130 bases, 120 bases, 110 bases, 100 bases, 90 bases, 80 bases, 70 bases, 60 bases, 50 bases, 40 bases, 30 bases, 20 bases, 10 bases, or 5 bases therefrom. Further, in some embodiments, at least one of the two PAM recognition sequences may be located at a position 200 bases or more away from the position of the base at the transcription start site, for example, 250 bases, 300 bases, 400 bases, 500 bases, 750 bases, 1,000 bases, 1,500 bases, 2,000 bases, 3,000 bases, 4,000 bases, 5,000 bases, 7,500 bases, 10,000 bases, 12,500 bases, or 15,000 bases or more away therefrom.

As another promoter editing method, there may also be used a method in which the region between two nicks generated by a Cas9 nickase is deleted, a method using base editing in which a target base in the genome is substituted with another base, and a method using a prime editor capable of deleting, inserting, or substituting in the genome any base sequence consisting of 1 to 20 bases, 20 to 50 bases, or 50 to 100 bases.

The number of different sequences included in the set of modified promoter sequences may be at least 5, 10, 20, 50, 100, 150, 200, 300, 500, 700, 1,000, 1,200, 1,500, 2,000, 3,000, 4,000, or 5,000. In order to sufficiently search for sequences affecting activity, the number of different sequences included in the set of modified promoter sequences is preferably 1,000 or more. Further, in order to sufficiently secure the number of different sequences included in the set of modified promoter sequences, the length of the original promoter sequence is preferably, for example, 1,500 bp or more, 1,600 bp or more, 1,700 bp or more, 1,800 bp or more, 1,900 bp or more, 2,000 bp or more, 2,100 bp or more, or 2,200 bp or more.

After the set of a plurality of modified promoter sequences has been generated, in some embodiments, the activity of each modified promoter sequence included in the generated set of modified promoter sequences is predicted by a machine learning model. For example, with respect to each generated base sequence, the 170 bases on the 3'-terminal side are obtained and input into the machine learning model. In some embodiments, 100 to 250 bases on the 3'-terminal side, for example, 100 bp, 110 bp, 120 bp, 130 bp, 140 bp, 150 bp, 160 bp, 170 bp, 180 bp, 190 bp, 200 bp, 210 bp, 220 bp, 230 bp, 240 bp, or 250 bp, may be obtained and input into the machine learning model. In some embodiments, a part of the sequence included in the 170 bases on the 3'-terminal side, for example, 100 to 169 bases, may be input. An appropriately trained machine learning model can output the strength (transcriptional activity) of a given base sequence as a core promoter.

In some embodiments, the activity of each modified promoter sequence included in the generated set of modified promoter sequences is visualized on a computer display. The visualization may be performed, for example, as shown in FIG. 8 or FIG. 9.

Then, from the prediction results of the machine learning model, by selecting a modified promoter sequence predicted to have the desired activity, a promoter sequence modified so as to have the desired activity can be obtained.

In some embodiments, the machine learning model may be a regression model trained to predict a gene expression-inducing activity from a promoter sequence, using, as training data, gene expression-inducing activity data of a plurality of promoter sequences in a plant cell. In some embodiments, the data of Jores et al. (2021) may be used for learning (Jores, T., Tonnies, J., Wrightsman, T. et al. Synthetic promoter designs enabled by a comprehensive analysis of plant core promoters. Nat. Plants 7, 842-855 (2021). https://doi.org/10.1038/s41477-021-00932-y). Accordingly, one aspect of the present disclosure relates to a method for generating a machine learning model for predicting a gene expression-inducing activity in a plant cell from a promoter sequence, the method comprising training a model using, as training data, gene expression-inducing activity data of a plurality of promoter sequences in a plant cell. In some embodiments, as the model to be trained, a transformer-based pre-trained foundation model may be used.

In some embodiments, in construction of the machine learning model, a deep learning model such as a transformer, particularly a model based on BERT, may be used. BERT (Bidirectional Encoder Representations from Transformers) is one of the machine learning models widely used in natural language processing (NLP). It was developed by Google in 2018, and remarkable performance was demonstrated in understanding and generation of text. Main features of BERT include (1) bidirectional contextual understanding, (2) use of a Transformer architecture, (3) pre-training and fine-tuning, and (4) diverse applicability. First, BERT is a "bidirectional" model and understands words in a given text in both left and right contexts. Whereas conventional models considered context only in one direction (from left to right, or vice versa), BERT can comprehensively understand the entire text. BERT is also built based on a neural network architecture called Transformer. Transformer captures relationships among words in input text using an "attention mechanism." This enables more complex and sophisticated text understanding. Further, BERT is "pre-trained" on a large amount of text data and has acquired a general understanding of language. Thereafter, by performing "fine-tuning" on a specific task (for example, sentiment analysis or question answering), BERT can be optimized for a particular application. BERT can be applied to various NLP tasks and is used in a wide range of fields such as text classification, question answering, sentiment analysis, and machine translation.

Two tasks are mainly used for training the BERT model. The MLM task is a task in which words are "masked" at random from input text and BERT is caused to predict the masked words. The main purpose of this task is to cause BERT to understand the meanings of words using context. Since bidirectional context is taken into account, the model predicts the masked words using information from the entire sentence. The NSP task causes BERT to determine whether two sentences are consecutive. The model is given one sentence (A) and another sentence (B), and is caused to predict whether B is a sentence that comes immediately after A. In this case, with a probability of one-half, B is an actual sentence following A, and with the remaining half, B is an unrelated sentence selected at random. The purpose of the NSP task is to cause BERT to understand relationships between sentences. This ability is particularly useful in tasks for which understanding continuity of sentences and the flow of meaning is important, such as question answering and summarization of text. By these tasks, BERT develops the ability to understand not only the word level but also the entire sentence and the relationships among a plurality of sentences. As a result, BERT becomes able to exhibit high performance in various natural language processing tasks.

In some embodiments, DNABERT, which is a transformer-based pre-trained foundation model, may be used for machine learning (Yanrong Ji, Zhihan Zhou, Han Liu, Ramana V. Davuluri, DNABERT: pre-trained Bidirectional Encoder Representations from Transformers model for DNA-language in genome, Bioinformatics, Volume 37, Issue 15, August 2021, Pages 2112-2120). DNABERT is a pre-trained bidirectional encoder representation capable of capturing a global understanding of genomic DNA sequences based on the context of upstream and downstream base sequences. In DNABERT, the NSP task used in BERT is not carried out, and learning is performed only by the MLM task. A certain proportion of a DNA sequence is masked, and the k-mer token at the masked site is predicted. The training data used for pre-training of DNABERT are DNA sequences sampled from the human genome. The present inventors demonstrated that, by fine-tuning a pre-trained foundation model such as DNABERT, a machine learning model for predicting a gene expression-inducing activity in a plant cell from a promoter sequence can be generated. The machine learning model according to the present disclosure can handle not only binary classification problems but also regression problems, and, as a result, can predict the level of gene expression-inducing activity.

One aspect of the present disclosure relates to a method for genome editing of a cell for regulating the expression level of a desired gene. In some embodiments, the method may be a method comprising obtaining a modified promoter sequence having the desired activity by the method for obtaining a modified promoter sequence modified so as to have a desired activity according to the present disclosure, providing a cell to be subjected to genome editing, and editing the genome of the cell so as to generate the modified promoter sequence.

In some embodiments, the cell is a plant cell, and examples thereof include cells of a wide range of plants including bryophytes, pteridophytes, gymnosperms, magnoliids, monocots, and eudicots (rosids I, rosids II, asterids I, asterids II, and outgroups thereof). More specific examples of plants include solanaceous plants such as tomato, sweet pepper, chili pepper, eggplant, tobacco, and torvum; cucurbits such as cucumber, pumpkin, melon, and watermelon; brassica vegetables such as cabbage, broccoli, Chinese cabbage, and kale; leafy and spice vegetables such as perilla, celery, parsley, and lettuce; alliums such as Welsh onion, onion, and garlic; other fruit vegetables such as strawberry and melon; taproot crops such as radish, turnip, carrot, and burdock; tuber crops such as taro, cassava, potato (Irish potato), sweet potato, and Chinese yam; cereals such as rice, corn, wheat, sorghum, barley, rye, Brachypodium distachyon, and buckwheat; legumes such as soybean, adzuki bean, mung bean, cowpea, common bean, peanut, pea, and broad bean; soft vegetables such as asparagus, spinach, and Japanese honewort; flowering plants such as lisianthus, rose, stock, carnation, and chrysanthemum; turfgrasses such as bentgrass and zoysiagrass; oil crops such as rapeseed, camelina, Brassica napus, Jatropha curcas, sesame, and perilla; fiber crops such as cotton, rush, and hemp; forage crops such as clover, dent corn, and alfalfa; deciduous fruit trees such as apple, pear, grape, and peach; citrus plants such as satsuma mandarin, orange, lemon, and grapefruit; and woody plants such as satsuki azalea, azalea, cedar, poplar, and Pará rubber tree.

One aspect of the present disclosure relates to a polynucleotide having promoter activity and having a sequence obtained by the method according to the present disclosure. Such a polynucleotide may, for example, have the sequence of SEQ ID NO: 2 or 4.

One aspect of the present disclosure relates to a method for producing a genome-edited plant in which the expression level of a desired gene is regulated. In some embodiments, the method may be a method comprising editing the genome of a desired plant cell by the method for genome editing of a cell for regulating the expression level of a desired gene according to the present disclosure, and obtaining a plant individual derived from the genome-edited cell. Further, one aspect of the present disclosure relates to a genome-edited plant produced by the production method according to the present disclosure.

In obtaining the genome-edited cell, a genome editing enzyme may be introduced into a plant tissue or cell in the form of DNA, RNP, or protein. Examples of the site for introduction of the genome editing enzyme into a plant include flower (egg cell, pollen, petal, and the like), stem (cambium, pith, cortex, and the like), leaf (including leaf primordium), root, shoot apex, axillary bud, flower bud, root tip, and protoplast.

The introduction method is not particularly limited, and may be selected as appropriate depending on the plant species and the cell/tissue to be introduced. Examples of the introduction method include an Agrobacterium method, a particle gun method, a whisker method, a nanopipette method, and virus-mediated nucleic acid delivery.

In some embodiments, in obtaining a plant individual derived from the genome-edited cell, the method may comprise, for example, a step (a) of culturing the genome-edited cell or a tissue containing the edited cell under appropriate culture conditions, a step (b) of proliferating the cells to form a callus (undifferentiated cell mass), a step (c1) of inducing a shoot from the callus, a step (c2) of directly forming a shoot from a tissue containing the edited cell, a step (d) of inducing roots in the shoot to regenerate a plant body, a step (e1) of obtaining a next-generation seed from the regenerated plant body, or a step (e2) of propagating the regenerated plant body by taking cuttings from a part thereof.

One aspect of the present disclosure relates to an information processing apparatus for predicting a promoter sequence modified so as to have a desired activity (see FIG. 11).

In some embodiments, the apparatus 010 may be an information processing apparatus comprising a sequence input unit 011 that receives input of an original promoter sequence to be modified, a modified sequence generation unit 012 that generates, based on the promoter sequence, a set of a plurality of modified promoter sequences that can be created by a genome editing technique, an activity prediction unit 013 that predicts, by a machine learning model, an activity of each modified promoter sequence contained in the generated set of modified promoter sequences, and a sequence selection unit 014 that selects a modified promoter sequence predicted to have the desired activity. The sequence input unit 011 may, for example, be connected to a communication device with an external network or an input device such as a keyboard. The sequence selection unit 014 may, for example, be connected to an output device such as a display or a printer, or a communication device with an external network. A person skilled in the art will be able to understand, in light of the disclosure of the present specification, the configuration necessary for the apparatus to implement the sequence obtaining method according to the present disclosure.

One aspect of the present disclosure relates to a non-transitory computer-readable medium storing instructions or a program.

In some embodiments, the computer-readable medium may be a computer-readable medium storing instructions or a program capable of causing, when the instructions or program are executed by a processor, the following steps to be performed: a step S100 of receiving input of an original promoter sequence to be modified, a step S110 of generating, based on the promoter sequence, a set of a plurality of modified promoter sequences that can be created by a genome editing technique, a step S120 of predicting, by a machine learning model, an activity of each modified promoter sequence contained in the generated set of modified promoter sequences, and a step S130 of selecting a modified promoter sequence predicted to have the desired activity. FIG. 12 shows an exemplary flowchart of such a program.

The step S110 of generating a set of a plurality of modified promoter sequences that can be created by a genome editing technique may further comprise a step S112 of comprehensively searching, from the input sequence, for positions cleavable by Cas9 (PAM sequences), a step S114 of combinatorially selecting all pairs of two among the searched cleavable positions (PAM sequences), and a step S116 of generating a set of modified promoter sequences by deleting the sequence between the two cleavage sites selected for all combinations. The step S120 of predicting an activity of each modified promoter sequence by a machine learning model may be performed using only the core promoter portion of each modified promoter sequence (150 to 200 bp on the 3'-terminal side, for example, a sequence of about 170 bp).

One aspect of the present disclosure relates to a computer program for predicting a promoter sequence modified so as to have a desired activity.

In some embodiments, the program may be a program for causing a computer to perform a function of receiving input of an original promoter sequence to be modified, a function of generating, based on the promoter sequence, a set of a plurality of modified promoter sequences that can be created by a genome editing technique, a function of predicting, by a machine learning model, an activity of each modified promoter sequence contained in the generated set of modified promoter sequences, and a function of selecting a modified promoter sequence predicted to have the desired activity.

FIG. 13 is a schematic diagram showing one exemplary aspect of the apparatus of the present disclosure. In FIG. 13, 100 denotes a computer, which includes a control unit 101, a storage unit 102, a peripheral device I/F unit 103, an input unit 104, a display unit 105, and a communication unit 106, these being connected by a bus 110. This configuration is illustrative, and various configurations may be adopted as appropriate.

The control unit 101 is composed of a CPU (Central Processing Unit), a ROM (Read Only Memory), a RAM (Random Access Memory), and the like. The CPU calls a program stored in the storage unit 102, the ROM, a recording medium, or the like into a work memory area on the RAM and executes the program, drives and controls each device connected via the bus 110, and realizes processing performed by the computer. The ROM is a nonvolatile memory and keeps programs such as a boot program and BIOS of the computer 100, data, and the like. The RAM is a volatile memory and temporarily keeps programs, data, and the like loaded from the storage unit 102, the ROM, a recording medium, or the like, and also includes a work area used by the control unit 101 when performing various types of processing. The storage unit 102 is, for example, an HDD (hard disk drive), and stores programs executed by the control unit 101 and various other data.

The peripheral device I/F (interface) unit 103 is a port for connecting the computer 100 and a peripheral device. The peripheral device I/F unit 103 is composed of USB, IEEE1394, RS-232C, or the like. The mode of connection with the peripheral device is not limited to wired or wireless. The input unit 104 includes an input device such as a keyboard, a pointing device such as a mouse, a numeric keypad, and the like, and performs operation instructions, operation commands, data input, and the like to the computer 100. The display unit 105 is a logic circuit or device driver for displaying video, images, or the like on a display device such as a liquid crystal panel. The input unit 104 and the display unit 105 may also be integrally configured as a touch display.

The communication unit 106 includes a communication control device, a communication port, and the like and is a wired or wireless communication interface that mediates communication with the network 120. The bus 110 is a communication path that mediates transmission and reception of control signals, data signals, and the like among the devices. The network 120 may further be connected to an external server 130 or network storage 140.

For example, by reading a program recorded on the computer-readable medium according to the present disclosure into the apparatus of FIG. 13, the computer can be caused to function as an information processing apparatus including a sequence input unit that receives input of an original promoter sequence to be modified, a modified sequence generation unit that generates, based on the promoter sequence, a set of a plurality of modified promoter sequences that can be created by a genome editing technique, an activity prediction unit that predicts, by a machine learning model, an activity of each modified promoter sequence contained in the generated set of modified promoter sequences, and a sequence selection unit that selects a modified promoter sequence predicted to have the desired activity.

Unless defined otherwise, all technical and scientific terms used herein have the same meanings as those commonly understood by one of ordinary skill in the art to which the present invention belongs. Although any methods and materials similar or equivalent to those described herein may be used in the practice or testing of the present invention, some possible and preferred methods and materials are now described. All publications mentioned herein are incorporated herein by reference for the purpose of disclosing and describing the methods and/or materials in connection with which the publications are cited. It is to be understood that the present disclosure supersedes the disclosure of any incorporated publication in the event of contradiction.

When a range of values is recited, it is to be understood that, unless the context clearly indicates otherwise, each intervening value to one-tenth of the unit of the lower limit between the upper and lower limits of that range is also specifically disclosed. Each smaller range between any stated value or intervening value in a stated range and any other stated value or intervening value in that stated range is also encompassed within the present disclosure. The upper and lower limits of these smaller ranges may independently be included in or excluded from the range, and each range in which either, neither, or both limit values are included in the smaller ranges is also encompassed within the present invention, subject to any specifically excluded limit value in the stated range. When the stated range includes one or both of the limit values, ranges excluding either or both of the included limit values are also included in the present invention. The term "about" with respect to a numerical value means within 5%.

The embodiments described herein are intended to be merely exemplary, and those skilled in the art will be able to make numerous variations and modifications without departing from the spirit of the present invention. Further, although certain variations and modifications may not lead to optimal results, they may nevertheless produce satisfactory results. All such variations and modifications are intended to be within the scope of the present invention as defined by the appended claims. Further, any combination of constituent elements disclosed herein, and expressions of the present disclosure converted among methods, apparatuses, systems, computer programs, data structures, recording media, and the like, are also effective as aspects according to the present disclosure. Therefore, the details described with respect to the methods of the present disclosure can also be applied to systems, computer programs, data structures, recording media, and the like.

### [EXAMPLES]

### Example 1: Construction of Machine Learning Model

A machine learning model for predicting a gene expression-inducing activity from a promoter sequence in a plant cell was constructed as follows. First, as source data for learning, the data shown in the paper by Jores et al. were obtained (Jores, T., Tonnies, J., Wrightsman, T. et al. Synthetic promoter designs enabled by a comprehensive analysis of plant core promoters. Nat. Plants 7, 842-855 (2021). https://doi.org/10.1038/s41477-021-00932-y). The outline of the dataset was about 70,000 datasets, each consisting of a 170-base DNA sequence and an expression intensity.

Further, DNABERT, which is a transformer-based pre-trained foundation model, was used for machine learning. DNABERT is reported, for example, in the paper by Ji et al. described above. DNABERT is mainly directed to handling binary classification problems, and has been shown, for example, to be capable of making a determination such as "whether a given base sequence has a splicing site." A binary classification problem is, in other words, a problem of classifying into two categories, such as 1 for the case that the sequence has a splicing site and 0 for the case that the sequence does not so. In contrast, a problem such as "predicting, for a given base sequence, the downstream gene expression level" is called a regression problem as opposed to a classification problem, and requires output of a continuous value. Accordingly, in order to handle continuous values with DNABERT, a class was added to the transformer and the model was trained.

### Example 2: Prediction and Classification of Promoter Strength by Trained Model

Promoter sequences are present upstream of genes and control the transcription amount (expression level) of genes located immediately downstream thereof in many cases. The expression level of a gene is considered to be controlled by the sequence of the promoter. The expression level of a downstream gene is defined as the strength of the promoter. Each promoter sequence is considered to exhibit its own specific strength.

As described in Example 1, a machine learning model for predicting transcriptional activity based on a base sequence was constructed. Using this model, a list was prepared in which the transcriptional activities of all genes of Arabidopsis thaliana were evaluated.

All promoters were classified into the following three groups based on the predicted transcriptional activity:
1. a "high expression group" predicted to exhibit high promoter strength;
2. a "low expression group" predicted to have promoter strength exhibiting very low or substantially no expression; and
3. a "medium group" exhibiting promoter strength intermediate therebetween.

### Example 3: Comparison Between Prediction by Model and Actual Gene Expression

Seven promoters were selected from the high expression group, eight promoters were selected from the medium group, and four promoters were selected from the low expression group, and artificial synthetic genes connected to a luciferase (LUC) gene were created.

The structure of the gene is as shown in FIG. 1. The promoters have 19 different sequence patterns. The sequence of the downstream LUC gene and the sequence of the upstream cauliflower mosaic virus (CaMV) 35S enhancer are common to each artificial gene.

A plasmid vector carrying this artificial gene was transfected into protoplasts collected from Arabidopsis thaliana leaves by the polyethylene glycol method. The protoplasts expressed the LUC gene at various expression levels. In order to evaluate the LUC expression level, luminescence intensity was measured using a plate reader. The relationship between the measurement results and the predicted promoter strength is shown in FIG. 2. A scatter plot shows the relationship between the measured values of the expression level of the LUC gene and the predicted values of promoter strength. The vertical axis indicates predicted transcriptional activity. The higher the value, the greater the expression level of the downstream gene is expected to be. The horizontal axis indicates measured values of log-transformed LUC expression intensity. It should be noted that the values were normalized by the expression level of the CaMV promoter, which was used as a positive control (P control).

From the results shown in FIG. 2, it can be seen that the measured values are divided into three groups, consistent with the groups based on the predicted values. Thus, a clear correlation was confirmed between the predicted values by the constructed machine learning model and the measured values. This figure shows the high predictability. In this figure, linear convergence means that the predicted values and the measured values coincide, indicating high prediction performance. When this prediction performance was evaluated by correlation coefficient R, R = 0.9296673 was obtained.

Next, based on the correlation shown in the scatter plot above, the LUC expression level corresponding to the predicted transcriptional activity was calculated by linear regression prediction. A comparison thereof is shown in FIG. 3 below.

The luminescence intensity of LUC was plotted on the vertical axis. It should be noted that the values are shown as relative values with the luminescence intensity of the P control taken as 1. As the P control, a cauliflower mosaic virus (CaMV) promoter known to have high transcriptional activity was used. The numbers of the 19 promoters are shown on the horizontal axis. Promoter Nos. 3 to 10 were classified into a "high expression" group in which high expression intensity was predicted, promoter Nos. 13 to 21 were classified into a "medium" group, and promoter Nos. 22 to 25 were classified into a "low expression" group. Black bar graphs indicate measured values (expression levels) of the LUC assay. White bar graphs indicate predicted values of expression intensity by the machine learning model. In FIG. 3, focusing on the black bar graphs, the "high expression" group exhibited relatively high expression levels, while the "low expression" group exhibited low expression levels. The "medium" group exhibited intermediate values.

Next, for individual promoters, it was examined how accurately the expression level could be predicted. Comparing the black bars and the white bars for each promoter, it can be seen that promoter Nos. 3, 4, 6, 7, 9, 10, 13, 15, 16, 20, and 24 were predicted with a high degree of agreement. On the other hand, in Nos. 21 and 22, the measured values were markedly greater than the predicted values. Conversely, in Nos. 5, 17, and 19, the predicted values were markedly greater than the measured values.

In summary, the constructed model was able to predict rough trends such as high expression, medium expression, and low expression. For low-expression promoters, the low-expression tendency could generally be predicted with high accuracy. In the high-expression group, expression could be predicted in the range of 50% to 280% of the measured values.

Thus, the machine learning model according to the present disclosure can predict a gene expression level based on the base sequence of a promoter.

### Example 4: Prediction and Verification of Promoter with Reduced Activity

Next, a method for reducing the transcriptional activity of a promoter was devised. Each promoter contains many PAM recognition sequences (NGG) for Cas9. These sequences were searched, and approximately several thousand possible editing patterns were listed.

A more specific procedure is as follows:
1. comprehensively searching for positions cleavable by Cas9 (PAM sequences) from the given base sequence;
2. selecting two sites therefrom;
3. cleaving at the two cleavage sites and simulating a new core promoter sequence that appears after repair;
4. estimating the strength of the simulated sequence as a core promoter;
5. carrying out the above steps for all conceivable pairs of cleavage sites; and
6. selecting one showing the lowest score (or, when selecting a promoter whose transcriptional activity is increased, the highest score) among all simulated core promoters.

By the foregoing, when it is desired to enhance or suppress expression level, it is possible to determine which cleavage sites should be selected. In this manner, by estimating the transcriptional activity of the promoter for the base sequence of each editing pattern, editing patterns capable of reducing the transcriptional activity of the promoter were searched for. The results are shown in FIG. 4. For promoter Nos. 3, 4, 5, 6, 9, 10, and 21, editing patterns capable of reducing promoter strength were searched for. Black triangular plots indicate predicted values of the scores of the LUC assay, calculated based on the predicted new promoter strength. As a result, transcriptional activity corresponding to an LUC expression level of about 14% to 1% relative to the original promoter was predicted. The deletion size of each promoter is shown in the table below.

**[Table 1]**

| Promoter# | Deletion size |
|---|---|
| 3 | 1524 |
| 4 | 1798 |
| 5 | 1520 |
| 6 | 1829 |
| 9 | 787 |
| 10 | 1414 |
| 21 | 444 |

Next, genes having these theoretical promoter sequences were artificially synthesized, similarly transfected into protoplasts, and the expression levels were measured with a plate reader. The results are shown in FIG. 5. Superimposed on FIG. 4, measured values of the LUC expression levels of promoters having the new sequences are shown as shaded bar graphs. Since the expression levels were very low, the vertical axis is shown on an enlarged scale. Promoter No. 5 was a missing value. For all six promoters for which measured values were obtained, marked decreases in expression level were observed, consistent with the predicted values. For example, the sequence of promoter No. 3 is 
CGGAAACTTGTCACTTCCTTTACATTTGAGTTTCCAACACCTAATCACGACAACAATCATATAGCTCTC
GCATACAAACAAACATATGCATGTATTCTTACACGTGAACTCCATGCAAGTCTCTTTTCTCACCTATAA
ATACCAACCACACCTTCACCACATTCTTCACT (SEQ ID NO: 1),
 and the predicted value of the transcriptional activity was 5.43, and the LUC expression level predicted by linear regression from this value was 111.5% relative to the P control. The measured value of the LUC expression level was 68.6%. In contrast, the edited sequence of promoter No. 3 is 
GAAACTGATTAGCTCCTATCAGTTCAGCAAACCACAAGCTGAAGAATCCAAGACTTGAGAAACAAATT
TACAAAAGCCCATGTTCCAATCAAAACTGTTACCAAACATCTGAAATAGATCTAAATGAGCGTTGGTA
TAATTGAAACTTACCGAAGGCCCACATTCTTCAC (SEQ ID NO: 2),
 and is generated when 1,824 bases between -1833 and -9 are trimmed. The predicted value of the transcriptional activity was 0.393, and the LUC expression level predicted by linear regression from this value was 7.98% relative to the P control. The measured value of the LUC expression level was 3.41% relative to the P control.

Thus, expression can be reduced by editing the base sequence of the promoter based on the prediction by the machine learning model according to the present disclosure.

### Example 5: Prediction and Verification of Promoters with Increased Activity

Finally, a method for increasing promoter activity was devised. As in the case of Example 4, among the promoter base sequences of conceivable editing patterns, those in which the predicted value of promoter strength after editing increased were searched for. The results are shown in FIG. 6. For promoter Nos. 13, 17, 22, 23, 24, and 25, those showing increased promoter strength were selected, and the predicted expression levels are shown by white circles. It was predicted that the LUC expression level would be 7.4- to 125-fold that of the original promoter. The deletion size of each promoter is shown in the table below.

**[Table 2]**

| Promoter# | Deletion size |
|---|---|
| 13 | 566 |
| 17 | 1336 |
| 22 | 1576 |
| 23 | 1760 |
| 24 | 1668 |
| 25 | 1281 |

Next, genes having these theoretical promoter sequences were artificially synthesized, similarly transfected into protoplasts, and the expression levels were measured with a plate reader. The results are shown in FIG. 7 below. Of the six promoters, expression levels of five promoters increased relative to those of the original promoters, but only No. 13 showed an increase in expression level exceeding the predicted value. For example, the sequence of promoter No. 13 is 
TCAAGCAATCATTATCGACTACGGTCGTTCGTTAAAGATCATGCATGTGCTTAGTGGCAATACCCTACG
CATCTTGATTCGTTACTGCGGCACGTGTCATGACCATGCACATGAATGATGATTAATGTTTAGTACATA
TAATGTTCACGCAAACGCATAGTGTTAGGAAA (SEQ ID NO: 3),
 and the predicted value of the transcriptional activity was 2.00, and the LUC expression level predicted by linear regression from this value was 6.94% relative to the P control. The measured value of the LUC expression level was 6.60% relative to the P control. In contrast, the edited sequence of promoter No. 13 is 
GAAACTTGAAAATCAAATCAGTGAGTCGCAAGTAAGACTTTGTGGTTGTTGTATCAGATTTCGCCGTG
CGCATCTTGATTCGTTACTGCGGCACGTGTCATGACCATGCACATGAATGATGATTAATGTTTAGTACA
TATAATGTTCACGCAAACGCATAGTGTTAGGAA (SEQ ID NO: 4),
 and the predicted value of the transcriptional activity was 4.18, and the LUC expression level predicted by linear regression from this value was 36.7% relative to the P control. The measured value of the LUC expression level was 69.5% relative to the P control.

Thus, expression can be increased by editing the base sequence of the promoter based on the prediction by the machine learning model according to the present disclosure.

### Example 6: Program for Changing Promoter Function by Genome Editing

The present inventors developed a program to be used for changing promoter function by genome editing according to the following procedure.

1. A base sequence X is input. For example, it is 2,000 bases from -1,995 to +5 around the transcription start site of the target gene.
2. PAM sequences are searched for in the base sequence X. (Cas9: NGG or Cas12a: TTTV sequence)
3. Based on the PAM sequences, a list of cleavage positions is stored in an array. Here, in the case of Cas9, the position 4 bases downstream of the PAM sequence serves as the cleavage position, and in the case of Cas12a, the position 18 bases downstream of the PAM sequence serves as the cleavage position. For example, about 50 cleavage positions are found in 2,000 bases.
4. Of n cleavage positions, the number of combinations in which two positions are designated is ₙC₂. For example, for 50 cleavage positions, there may be 1,250 combinations. For the ₙC₂ combinations, base sequences are generated in which the region between two cleavage positions is trimmed (deleted).
5. For each generated base sequence, the 170 bases on the 3'-terminal side are obtained and input into the learning model.
6. The learning model outputs the strength (transcriptional activity) of each of the given base sequences as a core promoter.
7. In summary, the following three types of information are obtained.
   A. Transcriptional activity of base sequence X
   B. Combinations of cleavage positions in which the promoter activity of a sequence generated by trimming the region between two cleavage positions is greater than A.
   C. Combinations of cleavage positions in which the promoter activity of a sequence generated by trimming the region between two cleavage positions is smaller than A.
8. For example, when increasing expression of the target gene, it is expected that an individual in which the region between the two cleavage sites is deleted can be obtained by simultaneously introducing two types of guide RNA targeting the cleavage positions obtained in 7.B. into a cell and performing genome editing by CRISPR-Cas9 or -Cas12a.

In particular, it should be noted that the simultaneous use of two different guide RNAs is intended to induce a small- to medium-scale deletion within the scope of category SDN1.

### Example 7: Visualization of Predicted Values of Promoter Activity

Ultimately, the program described in Example 6 yields a set of the positions of two guide RNAs and the predicted value of the new promoter activity of the sequence after deletion, but this is difficult to understand intuitively. Therefore, the present inventors also devised a method for visualizing these.

As a first visualization method, a method was devised for analyzing the overall picture of an input base sequence X. The vertical axis represents predicted promoter strength, and the horizontal axis represents the position in the base sequence. Each plot indicates the value obtained when the transcriptional activity is predicted by extracting a DNA sequence at a certain site with a 170-base window. For example, the point at position 0 on the horizontal axis indicates that 170 bases from the 5' end of the input base sequence X were extracted and the transcriptional activity was predicted. As a result, the transcriptional activity was 1.24 (since this 170-base sequence is not adjacent to a gene, it is unlikely to function as a core promoter, but it predicts that, if a gene were connected immediately downstream of this 170-base sequence, transcription would occur with a strength of 1.24). Accordingly, the point was plotted at the position (0, 1.24). Next, the window was slid by 1 base, and the base sequence from the 2nd base to the 171st base of the base sequence X was extracted and similarly predicted. As a result, the transcriptional activity was estimated to be 1.37. Accordingly, the point was plotted at the position (1, 1.37). By repeating this operation, all 1830 points were plotted (FIG. 8).

In FIG. 8, the point at the right end, (1829, 1.47), represents evaluation of the 170 bases immediately upstream of the gene. If it is possible to remove, the sequence between the 3' end and a point having a value higher than this value by genome editing, gene expression is expected to increase. Similarly, it is considered possible to decrease gene expression. By using this figure, it becomes clear, while obtaining an overall view of the entire sequence X, in which region guide RNAs should be designed to hybridize in order to obtain a desired expression level.

The above visualization method may be effective for roughly grasping the overall picture of the target sequence. On the other hand, it does not show the positional relationship between promoter strength and the two guide RNAs to be designed. In order to overcome this point, a visualization by another method was devised. FIG. 9 shown next is a visualization by another method.

A guide RNA designed at a position close to the target gene is called a proximal guide, and a guide RNA designed at a distant position is called a distal guide. Visualization was performed based on the positions of designable guide RNAs (positions of PAM sequences). However, only the range of 1,000 bases immediately upstream of the gene is shown.

The horizontal axis indicates the position in the sequence X of the base to which the distal guide hybridizes. That is, it indicates the distance between the transcription start site of the gene and the distal guide. Similarly, the vertical axis indicates the distance between the proximal guide and the transcription start site of the gene. Here, since the proximal guide is never designed farther away than the distal guide, half of the plots in FIG. 9 are hidden. At points on the line y = x, the proximal guide and the distal guide are both located at substantially the same distance from the gene and are close to each other. In this case, the deleted bases are few, so the editing is minimal and desirable. The 170 bases from the 3' end of the new sequence generated when the sequence between the proximal guide and the distal guide is deleted were extracted, and the transcriptional activity was predicted. The color of the plot is changed based on the predicted value. According to the color chart, bright gray indicates those predicted to have high transcriptional activity, and black indicates those predicted to have low transcriptional activity.

For example, the plot at the point (849, 17) will be described as an example. The position of the distal guide at this point is at a distance of 849 bases from the gene. Further, the position of the proximal guide is at a position 17 bases from the gene. A base sequence X' is created by deleting 832 bases between these two points from the base sequence X. When the 170 bases from the 3' end of the base sequence X' were extracted and the transcriptional activity was predicted, the value was 4.02. According to the color chart, a bright gray plot was placed. The figure was prepared by performing the above operation for all combinations of distal guides and proximal guides.

When it is desired to increase the expression level of this gene, points greater than the original transcriptional activity 2.34 may be selected, and the positions of guide RNAs to be designed may be read from the positions of the distal and proximal guides thereof. For example, when the point (849, 17) or the point (841, 29) is selected, high transcriptional activity is expected. Further, when the point (432, 110) or the point (271, 77) is selected, low transcriptional activity is expected. By visualizing in this manner, design of guide RNAs for realizing the structure of the target promoter may be simplified.

After the positions of the target sequences of the guide RNAs were obtained in this manner, the base sequences at those positions were obtained, and 20 bases or 23 bases were obtained to design guide RNAs. The sequences of these guide RNAs were evaluated and validated by a tool such as CRISPOR to evaluate high specificity, off-target effects, and the like. When appropriate guide RNAs were successfully designed, vectors carrying the guide RNAs were prepared by applying artificial DNA synthesis and a PCR method. Thereafter, genome editing can be performed according to a standard method.

### Example 8: Example of Soybean Gene Promoter

The training data for the machine learning model used promoter sequences of Arabidopsis thaliana, sorghum, and corn. Therefore, it was unclear whether the prediction system according to the present invention would function as expected also for promoter sequences of organisms other than these three species. Accordingly, an examination was conducted to increase transcriptional activity for a specific soybean gene. The results are shown in FIG. 10.

When the transcriptional activity of the 170-base sequence of the core promoter of a soybean gene was predicted, it was predicted to be 0.927494. Based on this value, the predicted LUC expression level was 1.26% of the positive control. This promoter sequence was artificially synthesized, introduced into protoplasts isolated from leaves of Arabidopsis thaliana, and subjected to a LUC assay. As a result, the value was 4.15%.

Based on this promoter, two promoter sequences after genome editing for increasing gene expression were created. These are referred to as edit1 and edit2, respectively. The transcriptional activity of edit1 was predicted to be 2.950119. Further, the LUC expression level was predicted to be 13.2%. The measured value of the LUC assay of this promoter was 19.0%.

The transcriptional activity of edit2 was predicted to be 2.432977. Further, the LUC expression level was predicted to be 7.27%. The measured value of the LUC assay of this promoter was 41.6%.

### Construction of Cas9 Expression Vector

A guide RNA expression cassette inducing the promoter sequences of edit1/edit2, for which increased expression had been confirmed in Arabidopsis thaliana protoplast experiments, a Cas9 gene expression cassette, and a hygromycin resistance gene expression cassette were constructed. Standard molecular biological techniques were used for construction of the expression vector. PCR amplification was performed using PrimeSTAR Max (Takara Bio Inc.). For purification of PCR products, NucleoSpin Gel and PCR Cleanup (Macherey-Nagel) was used. For assembly of fragments excised from plasmid DNA, NEB Golden Gate Assembly Kit (BsaI-HF v2) (New England Biolabs Japan Inc.) was used. Finally, the Cas9 gene expression cassette, the guide RNA expression cassette, and the hygromycin resistance gene expression cassette were inserted into the pTTK352 binary vector to obtain a genome editing tool expression vector.

### Cultivation of Soybean Plants

As plant material, soybean cultivar "Enrei" was used. In sowing, seed disinfection, humidity adjustment treatment, and sowing operation were performed in this order. As seed disinfection, the seeds were left to stand for 16 hours in a sealed container together with a beaker containing 20 mL of Haiter (Kao) and 1 mL of 12N hydrochloric acid. After seed disinfection, the seeds were left to stand at 4°C for 1 to 2 weeks in a sealed container together with a beaker containing 100 mL of deionized water as a humidity adjustment treatment. After the humidity adjustment treatment, the seeds were left to stand on MS medium as a sowing operation. In cultivation, the plants after sowing were cultivated in a growth chamber set at 25°C and a 16 h light period/8 h dark period.

### Isolation of Explants

Soybean plants about 1 day after the start of cultivation were used as explants. Under a stereomicroscope, the plants were cut in half, the hypocotyl was excised with a scalpel, and then each half was further cut vertically in half. The explants were wounded by tracing 10 times with a microbrush or a scalpel from the base of the hypocotyl toward the tip. Isolation was performed in a clean bench. The isolated explants were left to stand on a solid cocultivation medium prepared in accordance with the formulation described in a prior report (Tabei. 2012, Transformation Protocols [Plant Volume], Kagaku-Dojin).

### Gene Introduction into Explants

As Agrobacterium, strain EHA105 transformed with the genome editing tool expression vector was used. Infection with Agrobacterium and cocultivation were carried out in the order of preculture of Agrobacterium, preparation of an Agrobacterium suspension for infection, immersion of tissues in the suspension, and cocultivation. The media used were prepared in accordance with the formulation described in the prior report (Tabei. 2012, Transformation Protocols [Plant Volume], Kagaku-Dojin). As preculture of Agrobacterium, a glycerol stock of Agrobacterium was streaked on LB medium and cultured for 2 days at 28°C in darkness. As preparation of the Agrobacterium suspension for infection, the Agrobacterium after completion of preculture was resuspended in a liquid cocultivation medium and adjusted to OD600 = 0.8. In immersion of tissues in the suspension, the explants were immersed in the Agrobacterium suspension for infection for 20 minutes. In cocultivation, sterilized filter paper was placed on the solid cocultivation medium, the explants after completion of immersion were arranged thereon, and the explants were left to stand for 5 days at 25°C and a 16 h light period/8 h dark period.

### Redifferentiation

The medium used for shoot redifferentiation was prepared in accordance with the formulation described in the prior report (Tabei. 2012, Transformation Protocols [Plant Volume], Kagaku-Dojin). The explants after completion of cocultivation were washed with a liquid adventitious bud induction medium, inserted into a solid adventitious bud induction medium, and cultured for 2 weeks at 25°C and a 16 h light period/8 h dark period. Next, after elongated buds (non-transformants) were cut off, the explants were transplanted to a solid adventitious bud induction medium supplemented with 20 mg/L hygromycin and further cultured for 2 weeks. Thereafter, after browned portions were cut off with a scalpel, the explants were transplanted to an adventitious bud elongation medium supplemented with 20 mg/L hygromycin. The explants were transplanted to a fresh adventitious bud elongation medium every 2 weeks to obtain hygromycin-resistant shoots. The redifferentiated shoots were excised and transplanted to a rooting medium. After rooting, the shoots were potted up and cultivated in a closed greenhouse, and seeds were harvested.

### Expression Analysis in the Next Generation (T1 Generation)

After sowing the obtained seeds, DNA is extracted from seedlings, and individuals in which editing of edit1 or edit2 has occurred are examined and selected in the next generation (T1) by sequencing analysis. Then, RNA extraction and reverse transcription are performed from the selected individuals, and expression analysis is carried out by real-time PCR with respect to the soybean target gene having the promoter sequence of edit1 or edit2.

### Example 9: Genome Editing with One Target (One Guide RNA)

The graph of FIG. 14 shows results of in silico genome editing of the promoter sequence of the AT4G39130 gene of Arabidopsis thaliana with one target (one guide RNA). For six PAM sequences, sequences were created in which the 1- to 5-bp deletions normally introduced by cleavage by Cas9 were respectively added, and gene expression activity was predicted. As a result, it was found that the values should not greatly change from the intact sequence (without genome editing). This result indicates that a desired modification cannot be made simply by applying genome editing technology to a promoter region with one target. Thus, as a result of the extensive research unique to the present inventors, it was found that, with deletion of one target (normally resulting in a deletion of 1 to 5 bp), the expression intensity does not greatly change. This indicates that, with the one-target deletion initially expected by the present inventors, the expected result cannot be obtained, and this was an unexpected result.

On the other hand, as described in Example 4 and Example 5, when deletion of two targets was performed, it was predicted that the gene expression level would increase 6-fold or more or decrease to 1/8 or less, as compared with when deletion of one target was performed (see FIG. 15). The present inventors considered that this is because, when deletion of two targets is performed, a longer deletion (also referred to as Large Deletion) can be caused as compared with when deletion of one target is performed. Although it is academically known that deletion occurs when two gRNAs are used, the efficiency is low and applications are rare, and in light of the technical common general knowledge that the technique is not suitable for exploratory tasks (there being a teaching away), this was an extremely surprising discovery and resulted in an unexpectedly favorable effect.

### [Description of Reference Signs]

010: information processing apparatus
011: sequence input unit
012: modified sequence generation unit
013: activity prediction unit
014: sequence selection unit
100: computer
101: control unit
102: storage unit
103: peripheral device I/F unit
104: input unit
105: display unit
106: communication unit
110: bus
120: network
130: external server
140: database

## Claims

1. A method for obtaining a promoter sequence modified so as to have a desired activity, the method comprising:
providing an original promoter sequence to be modified;
generating, based on the promoter sequence, a set of a plurality of modified promoter sequences that can be created by a genome editing technique;
predicting, by a machine learning model, an activity of each modified promoter sequence contained in the generated set of modified promoter sequences; and
selecting a modified promoter sequence predicted to have the desired activity.

2. The method according to claim 1, wherein the desired activity is a gene expression-inducing activity higher than that of the original promoter sequence, or a gene expression-inducing activity lower than that of the original promoter sequence.

3. The method according to claim 1, wherein the promoter sequence is a promoter sequence of a plant cell.

4. The method according to claim 1, wherein the original promoter sequence comprises a core promoter sequence and an upstream sequence thereof.

5. The method according to claim 1, wherein the genome editing technique is a genome editing technique using a CRISPR/Cas system.

6. The method according to claim 5, wherein the set of a plurality of modified promoter sequences is generated by sequence deletion caused by cleavage induced by a combination of guide RNA sequences designed based on two PAM recognition sequences.

7. The method according to claim 1, wherein the set of modified promoter sequences comprises at least 1,000 different sequences.

8. The method according to claim 1, wherein the machine learning model is a regression model trained to predict a gene expression-inducing activity from a promoter sequence, using, as training data, gene expression-inducing activity data of a plurality of promoter sequences in plant cells.

9. The method according to claim 1, further comprising visualizing, on a computer display, the activity of each modified promoter sequence contained in the generated set of modified promoter sequences.

10. An information processing apparatus for predicting a promoter sequence modified so as to have a desired activity, comprising:
a sequence input unit that receives input of an original promoter sequence to be modified;
a modified sequence generation unit that generates, based on the promoter sequence, a set of a plurality of modified promoter sequences that can be created by a genome editing technique;
an activity prediction unit that predicts, by a machine learning model, an activity of each modified promoter sequence contained in the generated set of modified promoter sequences; and
a sequence selection unit that selects a modified promoter sequence predicted to have the desired activity.

11. A non-transitory computer-readable medium storing instructions, wherein, when the instructions are executed by a processor, the instructions cause the processor to perform the steps of:
receiving input of an original promoter sequence to be modified;
generating, based on the promoter sequence, a set of a plurality of modified promoter sequences that can be created by a genome editing technique;
predicting, by a machine learning model, an activity of each modified promoter sequence contained in the generated set of modified promoter sequences; and
selecting a modified promoter sequence predicted to have the desired activity.

12. A program for causing a computer to perform:
a function of receiving input of an original promoter sequence to be modified;
a function of generating, based on the promoter sequence, a set of a plurality of modified promoter sequences that can be created by a genome editing technique;
a function of predicting, by a machine learning model, an activity of each modified promoter sequence contained in the generated set of modified promoter sequences; and
a function of selecting a modified promoter sequence predicted to have the desired activity.

13. A method for genome editing of a cell for regulating an expression level of a desired gene, the method comprising:
obtaining a modified promoter sequence having a desired activity by the method according to claim 1;
providing a cell to be subjected to genome editing; and
editing the genome of the cell so as to generate the modified promoter sequence.

14. A method for producing a genome-edited plant in which an expression level of a desired gene is regulated, the method comprising:
editing the genome of a desired plant cell by the method according to claim 13; and
obtaining a plant individual derived from the genome-edited cell.

15. A machine learning model for predicting a gene expression-inducing activity in a plant cell from a promoter sequence, wherein the machine learning model is a regression model trained to predict a gene expression-inducing activity from a promoter sequence, using, as training data, gene expression-inducing activity data of a plurality of promoter sequences in a plant cell.

16. A method for generating a machine learning model for predicting a gene expression-inducing activity in a plant cell from a promoter sequence, comprising training a model using, as training data, gene expression-inducing activity data of a plurality of promoter sequences in a plant cell.

17. The method according to claim 16, wherein a transformer-based pre-trained foundation model is used as the model to be trained.
